# FASCICULE DE BREVET EUROPEEN

(11) **EP 2 301 612 B1**
(45) Date de publication et mention de la délivrance du brevet: **27.02.2019**
(21) Numéro de dépôt: 10176508.9
(22) Date de dépôt: 14.09.2010
(51) Int. Cl.: A61M 16/00, A61M 16/10

(54) **Procédé de suivi d'un débit de gaz consommé à partir d'un réservoir cryogénique**
Verfahren zur Nachverfolgung des Durchsatzes an verbrauchtem Gas ab einem Kryotank
Method for monitoring a flow of gas consumed from a cryogenic tank

(30) Priorité: 23.09.2009 FR 0956541
(43) Date de publication de la demande: 30.03.2011
(73) Titulaire: CRYOPAL, 77607 Bussy Saint-Georges (FR)
(72) Inventeur: Reymond, Christian, 95160, Montmorency (FR); Godart, Pierre M., 60500, Vineuil Saint Firmin (FR)
(74) Mandataire: De Cuenca, Emmanuel Jaime

(56) Documents cités:
- DE-A1- 10 315 245
- FR-A1- 2 916 889
- US-A- 3 707 078
- US-A1- 2003 189 492
- US-A1- 2009 020 015

## Description

L'invention concerne un procédé et un dispositif de suivi de la consommation de gaz à partir d'un réservoir cryogénique.

Plus précisément, l'invention peut être utilisée pour suivre le débit de gaz inhalé par un patient à partir d'un réservoir cryogénique pendant certains laps de temps, les autres paramètres du gaz étant par ailleurs soit fixés et connus, soit eux-mêmes suivis par d'autres méthodes. Dans le cas d'une oxygénothérapie, un patient inhale en général entre 0 et 8 litres de gaz par minute, le gaz se trouvant quasiment à la pression atmosphérique. Il peut s'agir d'une respiration naturelle ou assistée.

Les molécules de gaz respirées par le patient sont acheminées par un système de canalisations, par exemple une canule se terminant par un masque respiratoire. Le réservoir cryogénique permet de stocker ces molécules sous la forme d'un bain liquide, susceptible de produire, après vaporisation, un grand volume de gaz (environ 800 fois plus pour l'oxygène). Ce bain est surmonté d'un ciel gazeux. Des moyens mécaniques permettent d'assurer la détente du gaz et le contrôle du débit inhalé.

Plusieurs méthodes existent pour suivre le débit gazeux consommé. Le suivi du débit gazeux consommé est réalisé généralement en mesurant le débit circulant dans la canule à l'aide de débitmètres. Ceci suppose d'ajouter des équipements éventuellement encombrants, coûteux et créant une perte de charge supplémentaire. Ceci peut diminuer marginalement la valeur du débit et crée de l'encombrement. En outre, dans le cas d'un appareil d'oxygénothérapie, si l'on veut ajouter un système pour contrôler la respiration du patient, il faudra le placer entre le patient et le débitmètre, ce qui crée de l'encombrement. Ces équipements doivent être compatibles avec l'oxygène, ce qui peut augmenter leur coût.

Le document FR 2916 889 A1 décrit un dispositif de surveillance à distance de cuves d'oxygène liquide comprenant une jauge de mesure de la quantité d'oxygène restant dans la cuve. En fonction de la mesure de la quantité d'oxygène restant un moyen de traitement détermine une date d'intervention sur la cuve.

Le document US 3,707,078 décrit un dispositif de conversion d'oxygène liquide d'un réservoir cryogénique en oxygène gazeux. La quantité de liquide dans le réservoir est mesurée par une sonde capacitive.

Aucun des dispositifs de ces documents ci-dessus ne permet de déduire le débit de gaz réellement consommé. Ces dispositifs mesurent un niveau de liquide mais sont incapables d'en donner le débit de gaz consommé (c'est-à-dire une consommation de gaz en dehors des soutirages de liquide).

Un but de la présente invention est de pallier tout ou partie des inconvénients signalés ci-dessus.

A cette fin, l'invention peut concerner un procédé de suivi d'un débit de gaz consommé par un patient, ledit gaz étant acheminé par un système de canalisations depuis un réservoir cryogénique comprenant au moins un bain dudit gaz stocké sous forme liquide dans un récipient, ledit bain possédant un certain volume correspondant à une certaine hauteur, caractérisé en ce qu'il comprend au moins les étapes suivantes :
a) A plusieurs instants pendant une période de temps pré-déterminée, on mesure ladite hauteur avec une précision relative d'au moins un dix-millième (1/10000) pour obtenir une série H de mesures de hauteur ; et
b) On utilise des valeurs comprises dans la série H obtenue à l'étape a) pour calculer une série D de quantités dudit gaz assimilables à des débits, correspondant à des variations de volume dudit bain au cours de ladite période prédéterminée.

Le débit de gaz à suivre provient d'un réservoir cryogénique via un système de canalisation. Par « suivi », ou « observance », on veut dire qu'on regarde ce débit, qu'il soit nul ou non, c'est à dire que le patient respire ou non le gaz en question, qu'il soit ou non connecté au réservoir. Le but est de connaître les débits consommés sous forme gazeuse ou soutirés du réservoir sous forme liquide, pendant certains intervalles de temps donnés s'inscrivant dans une période prédéterminée, par exemple par tranches de 15 minutes pendant 24 heures. La présentation de ces résultats, par exemple sous forme d'un histogramme permet de suivre aisément l'observance d'un traitement par le patient.

En fonction du système inhalation, il est possible que d'autres débits de gaz viennent s'ajouter au débit suivi. On peut penser à de l'air extérieur qui entrerait au niveau du masque d'inhalation. On peut penser aussi à un flux gazeux provenant d'un autre réservoir cryogénique, auquel cas son débit peut être suivi en appliquant le procédé selon l'invention à ce réservoir.

Par réservoir cryogénique, on entend tout réservoir apte et conçu pour stocker des gaz sous une forme liquéfiée. Dans le contexte de l'invention, le réservoir n'a pas vocation à demeurer au même endroit. Il est par exemple monté sur roulettes pour pouvoir être déplacé chez le patient. A priori, il n'a pas pour autant vocation à être bougé quand le patient est connecté.

Les molécules consommées sont stockées dans le réservoir cryogénique sous forme d'au moins un bain de liquide possédant un certain volume lié à une certaine hauteur. Ce bain se vaporise au fur et à mesure de la consommation de gaz (par exemple par un patient). Le débit moyen de liquide consommé peut être très faible comparé au débit gazeux moyen. S'il s'agit d'une oxygénothérapie, on a affaire à de l'oxygène très pur et le débit liquide consommé est alors environ 800 fois plus faible que le débit d'oxygène gazeux dans des conditions normales. La consommation de liquide se traduit donc par une baisse extrêmement lente du niveau du bain. Par exemple, pour un bain liquide cylindrique de 50 cm de diamètre, un débit gazeux typique de 1 litre/min correspond à une baisse moyenne de niveau d'environ 6.5 µm/min (micromètre/minute). La mesure de ces variations est donc difficile. En outre, la baisse du niveau du bain liquide est susceptible d'être perturbée par un certain nombre de facteurs (perturbation des mesures, résolution du système d'acquisition, pertes statiques ou naturelles du réservoir, etc.). Il n'est donc pas évident de pouvoir estimer le débit de gaz consommé en observant l'évolution de la hauteur de liquide dans le réservoir.

Les inventeurs ont cependant établi qu'il était possible de mesurer la hauteur de bain avec la précision relative voulue, de l'ordre de 5 µm pour une hauteur de bain typique de 50 cm, soit une précision relative de un dix-millième (1/10000). Ceci exclut notamment des mesures faites à l'aide de capteurs de pression ordinaires. La précision relative des mesures de hauteur est préférentiellement d'au moins un cent-millième (1/100000). Les inventeurs ont montré, que de façon surprenante et inattendue, on peut relier ces mesures de hauteur de liquide de manière pertinente à un débit de gaz consommé, comme par exemple le débit de gaz respiré par un patient. Plus précisément, ils ont montré que, malgré la vaporisation et les différentes capacités tampons présentes entre le bain liquide et le masque respiratoire, il existe une linéarité suffisante entre les variations de la hauteur du liquide et les variations du débit moyen de gaz consommé sur quelques secondes à quelques minutes.

A l'étape b), on utilise des mesures réalisées à l'étape a). Ceci ne veut pas dire que les étapes a) de mesures et b) d'utilisation des mesures soient successives. Elles peuvent être plus ou moins concomitantes, selon que les mesures faites sont utilisées immédiatement ou après un certain laps de temps. Dans ce cas, il peut être nécessaire de mémoriser les mesures et/ou les résultats de calculs.

A l'étape b) on calcule des quantités qui sont en réalité des estimations indirectes du débit de gaz consommé, car ces estimations sont réalisées en observant le bain liquide. Ces quantités calculées sont de préférence des débits gazeux (en mètre cube pris dans certaines conditions de température et de pression par minute, ou toute autre unité équivalente). Elles peuvent être des débits liquides (en mètre par minute, ou toute autre unité équivalente). Elles peuvent être des volumes ou des masses, s'il s'y attache implicitement une notion de durée pendant laquelle ce volume ou cette masse de molécules ont été consommées. En fait, ces quantités sont donc toujours, implicitement ou explicitement, des débits.

Les inventeurs ont en outre déterminé qu'on peut effectuer le calcul d'un de ces débits à partir de deux mesures de la hauteur du bain effectuées à un intervalle de temps donné, et que la moyenne, sur une certaine période de temps des débits ainsi calculés est représentative du débit moyen de gaz réel consommé pendant cette période. Il également possible de partir des mesures de hauteur au début et à la fin de ladite période. On obtient ainsi plus directement un débit moyen consommé pendant cette période.

Comme on mesure le niveau de liquide à l'étape a), on voit qu'un avantage de l'invention est de permettre la planification des tournées de distribution et de réapprovisionnement par camion de ce liquide et la télésurveillance du niveau dans le réservoir.

Les mesures de débit gazeux et/ou liquide peuvent être exploitées localement ou bien envoyées, par exemple par des moyens sans fil, pour un traitement à distance. De ce fait, à nouveau, il peut être nécessaire de stocker les données.

Par ailleurs, selon des modes de réalisation particuliers, l'invention peut comporter l'une ou plusieurs des caractéristiques suivantes :
- à l'étape b), on calcule chaque quantité de la série D selon un algorithme comprenant les étapes suivantes :
   i) on convertit deux mesures de hauteur (3) appartenant à ladite série H, correspondant à deux instants de mesure, en deux volumes dudit bain (2) ;
   ii) on divise la différence entre les deux volumes obtenus au i) par la durée séparant lesdits instants de mesure afin d'obtenir le débit de liquide consommé pendant la durée séparant lesdits instants ;
   iii) si ledit débit de liquide obtenu au ii) est inférieur à un seuil prédéterminé, on multiplie ledit débit de liquide par un facteur d'expansion donné pour obtenir ladite quantité dudit gaz consommé; et
   iv) si ledit débit de liquide obtenu au ii) est supérieur ou égal audit seuil prédéterminé, la quantité dudit gaz consommée sera considérée nulle.
- ledit gaz provenant dudit réservoir cryogénique comprend majoritairement de l'oxygène. Par majoritairement, on entend au moins 50% en volume (à l'état gazeux), préférentiellement au moins 90% et encore plus préférentiellement au moins 99%.
- à l'étape a), on mesure ladite hauteur à l'aide d'au moins une sonde de type capacitif.
- à l'étape a), on mesure ladite hauteur (3) à intervalles de temps de temps réguliers compris entre 0.1 seconde et 1 heure, préférentiellement entre 1 et 60 secondes et encore plus préférentiellement toutes les 10 secondes.
- le procédé comprend en outre une étape c) où l'on envoie par ondes hertziennes tout ou partie de la série D de quantités obtenue à l'étape b) à une passerelle GPRS, puis à un serveur distant par signaux GPRS.
- à l'étape c), on envoie tout ou partie de la série D de quantités obtenue à l'étape b) par ondes hertziennes en dehors des instants où on effectue des mesures à l'étape a).

En pratique, on peut mesurer la hauteur du bain liquide à intervalles de temps donnés et on obtient une série H de valeurs. La conversion de cette série H en une série D de valeurs de débit peut se faire par tout algorithme de calcul approprié. En général, on convertit d'abord la hauteur mesurée en volume de liquide, connaissant la relation qui lie pour tout récipient la hauteur de liquide au volume de liquide. En divisant une variation de volume liquide par la durée pendant laquelle le volume a varié, on obtient un débit liquide. Si ce débit liquide est supérieur ou égal à un seuil pré-déterminé, par exemple 0.1 litre/minute, on considèrera que le volume de liquide correspondant (c'est à dire la variation de volume du bain liquide) a été soutiré directement sous forme de liquide. Ceci peut se produire par exemple si l'utilisateur décide de transvaser du gaz sous forme liquide du réservoir cryogénique vers un réservoir auxiliaire portatif. Dans ce cas, on considèrera le débit gazeux consommé comme nul. Dans le cas contraire, si le débit de liquide est inférieur à ce seuil pré-déterminé, on le convertit en un débit gazeux équivalent, calculé à une pression et une température de référence. Pour ce faire, on le multiplie par le facteur d'expansion entre le liquide et le gaz. La méthode permet aussi de suivre les soutirages de liquide. Ces quantités, si on désire les suivre, pourront être présentées sous la forme de débits gazeux équivalents, en les multipliant le facteur d'expansion entre le liquide et le gaz.

On voit donc qu'un avantage important du procédé de suivi selon l'invention est de permettre également de suivre les quantités consommées sous la forme d'extractions de liquide, sans ajouter d'équipements de mesure supplémentaires. Ceci permet de justifier de la déambulation, ce qui peut- être une des conditions du remboursement du traitement d'oxygénothérapie par les caisses d'assurance maladie ou mutuelles.

Il est également possible de corriger les débits obtenus de l'effet de l'évaporation naturelle du liquide dans le réservoir, qui peut comprendre un échappement au niveau du ciel gazeux. Ceci peut se faire en retranchant aux débits obtenus une valeur prédéterminée ou calculée. Cette évaporation est en général faible au regard de la consommation effective. La correction n'est donc pas indispensable. Les quantités évaporées peuvent d'ailleurs être récupérées par un économiseur, c'est-à-dire un dispositif permettant de les renvoyer vers le patient.

Les sondes de type capacitif comprennent en général deux électrodes allongées, plongeant dans le bain liquide. L'une d'entre elles peut être une paroi du réservoir cryogénique. La hauteur de liquide modifie la nature du diélectrique entre les deux électrodes et donc la capacité électrique de la sonde considérée comme un condensateur. Plus le liquide est haut, plus la capacité est élevée. La mesure de cette capacité permet de remonter à la hauteur de liquide. En pratique, la sonde se présente de préférence sous la forme de deux tubes concentriques formant des électrodes. La capacité électrique est en général convertie en signaux de fréquence proportionnelle à la capacité ou en signaux rectangulaire dont le temps haut ou bas est proportionnel à la capacité par un circuit électronique.

Les inventeurs ont montré que ces sondes capacitives sont adaptées à la mesure de variations infimes de niveau de liquide et permettent d'en déduire le débit gazeux consommé par exemple par inhalation par un patient. Typiquement, la valeur de la capacité électrique varie entre 100 et 150 pF (picofarad). Ces sondes ont l'avantage de présenter un coût modeste et une consommation faible rendant leur mise en oeuvre aisée, en comparaison par exemple d'une pesée du réservoir cryogénique. La pesée ultra-précise d'un réservoir de quelques dizaines de kg serait extrêmement difficile et probablement coûteuse à mettre en oeuvre.

La passerelle GPRS reçoit les ondes hertziennes en provenance de l'émetteur et les ré-émet vers un serveur distant sous forme de signaux GPRS. Le serveur distant est en général un ordinateur ou un téléphone mobile, ou tout terminal équivalent possédant des fonctions de traitement de l'information. Les données sont en général présentées sous la forme d'histogrammes ou de courbes figurant le débit gazeux moyen par périodes de temps.

Les hauteurs mesurées ou les débits calculés peuvent éventuellement être mémorisés, par exemple au niveau d'un convertisseur, pendant un temps suffisant pour permettre l'envoi par ondes hertzienne à des moments où on n'effectue pas de mesure de hauteur par la sonde capacitive. Ainsi, on évite que les ondes hertziennes dues à l'émetteur ne perturbent la mesure par la sonde capacitive.

L'invention concerne également un dispositif de suivi de l'observance par un patient d'un traitement comprenant l'inhalation d'un gaz acheminé par un système de canalisations depuis un réservoir cryogénique comprenant un bain dudit gaz stocké sous forme liquide, ledit bain possédant un certain volume correspondant à une certaine hauteur, caractérisé en ce qu'il comprend :
- au moins une sonde de mesure apte et destinée à produire des signaux primaires représentatifs de ladite hauteur à différents instants ; et
- un convertisseur numérique destiné à convertir lesdits signaux primaires en signaux secondaires représentatifs de débits dudit gaz inhalés par ledit patient.

La sonde placée dans le réservoir produit des signaux que nous appelons « primaires » pour les distinguer d'autres signaux. Le convertisseur numérique est un dispositif électronique permettant la conversion de ces signaux primaires représentant une série de mesures de hauteur de bain liquide en signaux secondaires représentant une série de valeurs de débit. Il comprend en général un étage de mise en forme électronique des signaux de capacité en signaux fréquentiels et un micro-contrôleur. Il peut aussi assurer une fonction de mémorisation des données.

Par ailleurs, selon des modes de réalisation particuliers, l'invention peut comporter l'une ou plusieurs des caractéristiques suivantes :
- ladite sonde de mesure est de type capacitif.
- ledit convertisseur numérique est solidaire dudit réservoir cryogénique et connecté électriquement à ladite sonde de mesure.
- ledit dispositif comprend un émetteur connecté électriquement audit convertisseur numérique et apte et destiné à transformer lesdits signaux secondaires en signaux ternaires hertziens.
- ledit dispositif comprend une passerelle GPRS séparée dudit réservoir cryogénique et apte et destinée à recevoir lesdits signaux ternaires hertziens et à les ré-émettre sous forme de signaux quaternaires de type GPRS.
- ledit dispositif comprend au moins un serveur distant apte et destiné à recevoir et traiter lesdits signaux quaternaires de type GPRS.

Le convertisseur est de préférence solidaire du réservoir cryogénique. C'est en général une carte électronique de petites dimensions. Il comprend une partie pour le traitement du signal de capacité et sa transformation en général en un signal de fréquence. Une autre partie peut gérer la liaison avec un éventuel émetteur destiné à envoyer les signaux de hauteur de bain liquide ou de débit gazeux calculé à une passerelle GPRS. Les communications se font de préférence par ondes hertziennes entre l'émetteur et la passerelle GPRS, plutôt que par wifi pour des raisons de consommation électrique et de disponibilité de réseau. Les communications entre la passerelle GPRS et le ou les serveurs distants se font plutôt par signaux GPRS, plutôt que par SMS pour des questions de coût.

De manière autonome, sans transmettre d'information par GPRS, on peut prévoir que le dispositif selon l'invention fournisse l'information des débits mesurés et enregistrés localement à un technicien qui les récupère lors de sa tournée. Dans ce cas, le système conserve des avantages :
- justifier de la déambulation (cela permet de prouver des consommations de liquide par soutirage au profit d'un réservoir portatif distinct),
- permettre un suivi de l'observance (le technicien se substitue à la passerelle GPRS),
- le coût : pas de passerelle, pas de coût de communication induit.

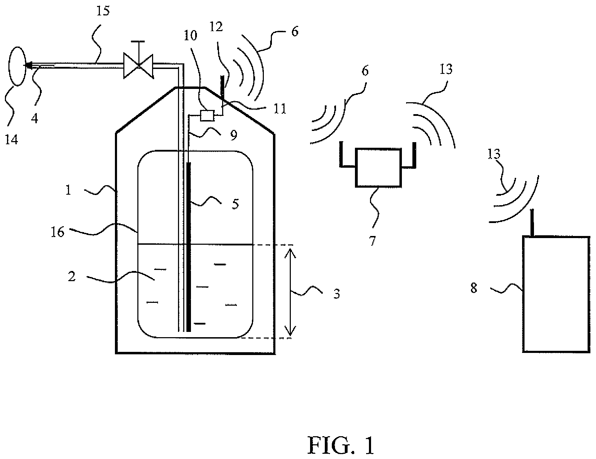

D'autres particularités et avantages apparaîtront à la lecture de la description ci-après, faite en référence à la figure 1 qui représente un exemple de dispositif selon l'invention.

Sur la figure 1 est représenté un réservoir cryogénique 1 placé chez le patient. Ce réservoir peut être monté sur roulettes (non représentées) afin de pouvoir être déplacé. Il comprend au moins un bain d'oxygène liquide 2 de hauteur 3. Ce bain liquide 2 a par exemple une forme cylindrique de diamètre 50 cm. Par une canule 15 et un masque respiratoire 14, cet oxygène peut être consommé par inhalation par un patient sous la forme d'un débit gazeux 4. De l'air extérieur peut se mélanger à l'oxygène au niveau du masque 14. Suivre l'observance par le patient de son traitement nécessite de suivre le débit gazeux 4 d'oxygène qu'il consomme.

Le réservoir comprend une sonde capacitive 5 qui plonge dans le bain 2 d'oxygène liquide. Cette sonde est telle qu'elle présente une capacité électrique qui dépend de la hauteur 3 de liquide. Cette hauteur est mesurée à intervalles de temps de 10 secondes. Il en résulte des signaux primaires électriques 9 qui représentent une série H de hauteurs. Ceux-ci arrivent à un convertisseur 10 qui les transforme en signaux secondaires 11 représentant une série D de débits gazeux estimés. Ces signaux secondaires 11 sont envoyés à un émetteur 12 et envoyés sous forme de signaux ternaires 6 hertziens à une passerelle GPRS 7. Cette passerelle 7 les capte et les ré-émet sous forme de signaux quaternaire 13 de type GPRS. Ceux-ci sont captés par un serveur ou un ordinateur distant 8.

Le passage de la série H (hauteurs) à la série D (débits gazeux estimés) se fait en considérant deux hauteurs mesurées successivement. Ces hauteurs, multipliées par la section du bain liquide (par exemple environ 0.2 mètres carrés) donnent deux volumes successifs. La différence entre ces deux volumes successifs, divisée par l'intervalle de temps entre les deux mesures, soit 10 secondes, donne un débit de liquide. Si ce débit est supérieur ou égal à 0.1 litre/minute, on considère qu'il correspond à un soutirage d'oxygène liquide et non à une consommation de gaz par inhalation d'oxygène gazeux. Le débit gazeux est alors considéré nul. Si au contraire le débit liquide est inférieur à 0.1 litre/minute, on multiplie le débit liquide par 800, c'est à dire le facteur d'expansion entre l'oxygène liquide et l'oxygène gazeux à 20°C et 1 atmosphère (101325 Pa ou pascal). On obtient alors un débit gazeux équivalent. Ces débits sont ensuite intégrés par périodes de 15 minutes au niveau de l'électronique sur le réservoir cryogénique 1, en vue de leur traitement ultérieur par un serveur distant 8 (téléphone, ordinateur). On obtient ainsi, de préférence graphiquement, un suivi du débit d'oxygène gazeux inhalé par le patient par période de 15 minutes. La durée de ces périodes peut bien sûr être ajustée en fonction des circonstances et des besoins spécifiques liés à tel ou tel type de traitement et le suivi qu'on veut en faire.

On comprend donc que les avantages de l'invention sont notamment :
- de ne pas ajouter de débitmètre gazeux sur la canule allant du réservoir au patient, ce qui permet de diminuer l'encombrement et les pertes de charge,
- d'obtenir des mesures de débit de gaz consommé précises et modulables dans le temps, adaptables au type de suivi du débit désiré,
- de pouvoir alimenter la sonde et l'électronique liée par pile (faible consommation électrique), ce qui renforce la sécurité au niveau du réservoir cryogénique,
- d'ergonomie (communication sans fil), identification à distance de l'état de fonctionnement du réservoir,
- d'obtenir également une mesure des quantités soutirées sous forme liquide,
- de suivre l'observance générale du traitement par le patient,
- de disposer d'une évaluation des pertes par évaporation.

## Revendications

1. Procédé de suivi d'un débit de gaz (4) consommé à partir d'un réservoir cryogénique (1) stockant le gaz sous forme liquéfié, ledit gaz consommé étant acheminé par un système de canalisations (15) depuis le réservoir cryogénique (1), le réservoir (1) comprenant au moins un bain (2) dudit gaz stocké sous forme liquide, le bain (2) possédant un certain volume correspondant à une certaine hauteur (3) de liquide, **caractérisé en ce qu'**il comprend au moins les étapes suivantes :
a) à plusieurs instants pendant une période de temps pré-déterminée, on mesure ladite hauteur (3) de liquide avec une précision relative d'au moins un dix-millième (1/10000) pour obtenir une série H de mesures de hauteur ; et
b) on utilise des valeurs comprises dans la série H obtenue à l'étape a) pour calculer une série D de quantités dudit gaz assimilables à des débits, correspondant à des variations de volume dudit bain (2) au cours de ladite période prédéterminée.

2. Procédé selon la revendication 1, **caractérisé en ce que**, à l'étape b), on calcule chaque quantité de la série D selon un algorithme comprenant les étapes suivantes :
i) on convertit deux mesures de hauteur (3) appartenant à ladite série H, correspondant à deux instants de mesure, en respectivement deux volumes dudit bain (2) ;
ii) on divise la différence entre les deux volumes obtenus au i) par la durée séparant lesdits instants de mesure afin d'obtenir le débit de liquide consommé pendant la durée séparant lesdits instants ;
iii) si ledit débit de liquide obtenu au ii) est inférieur à un seuil prédéterminé, on multiplie ledit débit de liquide par un facteur d'expansion donné pour obtenir ladite quantité dudit gaz consommé par le patient ; et
iv) si ledit débit de liquide obtenu au ii) est supérieur ou égal audit seuil prédéterminé, la quantité dudit gaz consommée est considérée nulle.

3. Procédé selon l'une quelconque des revendications 1 ou 2, **caractérisé en ce que** ledit gaz (4) provenant dudit réservoir cryogénique (1) comprend majoritairement de l'oxygène.

4. Procédé selon l'une quelconque des revendications 1 à 3, **caractérisé en ce que**, à l'étape a), on mesure ladite hauteur (3) à l'aide d'au moins une sonde (5) de type capacitif.

5. Procédé selon l'une quelconque des revendications 1 à 4, **caractérisé en ce que**, à l'étape a), on mesure ladite hauteur (3) à intervalles de temps de temps réguliers compris entre 0,1 seconde et 1 heure, préférentiellement entre 1 et 60 secondes et encore plus préférentiellement toutes les 10 secondes.

6. Procédé selon l'une quelconque des revendications 1 à 5, **caractérisé en ce qu'**il comprend en outre une étape c) où l'on envoie par ondes hertziennes (6) tout ou partie de la série D de quantités obtenues à l'étape b) à une passerelle GPRS (7), puis à un serveur distant (8) par signaux GPRS (13).

7. Procédé selon la revendications 6, **caractérisé en ce qu'**à l'étape c), on envoie tout ou partie de la série D de quantités obtenue à l'étape b) par onde hertziennes (6) en dehors des instants où on effectue des mesures à l'étape a).

8. Dispositif de suivi de la consommation de gaz à partir d'un réservoir cryogénique (1) stockant le gaz sous forme liquéfiée en particulier pour l'observance par un patient d'un traitement comprenant l'inhalation du gaz consommé (4), le gaz étant consommé via un système de canalisations (15) depuis le réservoir cryogénique (1) comprenant un bain (2) dudit gaz stocké sous forme liquide, ledit bain possédant un volume déterminé correspondant à une hauteur déterminée (3) de liquide, **caractérisé en ce qu'**il comprend :
- au moins une sonde de mesure (5) apte et destinée à produire des signaux primaires (9) représentatifs de ladite hauteur (3) de liquide du bain (2) à différents instants ; et
- un convertisseur numérique (10) configuré pour convertir lesdits signaux primaires en signaux secondaires (11) représentatifs de débits dudit gaz équivalents consommés.

9. Dispositif selon la revendication 8, **caractérisé en ce que** ladite sonde de mesure (5) est de type capacitif.

10. Dispositif selon l'une quelconque des revendications 8 ou 9, **caractérisé en ce que** :
- ledit convertisseur numérique (10) est solidaire dudit réservoir cryogénique (1) et connecté électriquement à ladite sonde de mesure (5) ; et
- ledit dispositif comprend un émetteur (12) connecté électriquement audit convertisseur numérique (10) et apte et destiné à transformer lesdits signaux secondaires (11) en signaux ternaires (6) hertziens.

11. Dispositif selon la revendication 10, **caractérisé en ce que** :
- ledit dispositif comprend une passerelle GPRS (7) séparée dudit réservoir cryogénique (1), apte et destinée à recevoir lesdits signaux ternaires (6) hertziens et à les ré-émettre sous forme de signaux quaternaires (13) de type GPRS ; et
- ledit dispositif comprend au moins un serveur distant (8) apte et destiné à recevoir et traiter lesdits signaux quaternaires (13) de type GPRS.

## Patentansprüche

1. Verfahren zum Nachverfolgen eines Durchsatzes (4) an verbrauchtem Gas abeinem Kryobehälter (1), der das Gas in verflüssigter Form speichert, wobei das verbrauchte Gas von einem Rohrleitungssystem (15) aus dem Kryobehälter (1) befördert wird, wobei der Behälter (1) mindestens ein Bad (2) des in flüssiger Form gespeicherten Gases umfasst, wobei das Bad (2) ein gewisses Volumen aufweist, das einer gewissen Flüssigkeitshöhe (3) entspricht, **dadurch gekennzeichnet, dass** es mindestens die folgenden Schritte umfasst:
a) die Flüssigkeitshöhe (3) wird zu mehreren Zeitpunkten während eines vorbestimmten Zeitraums mit einer relativen Genauigkeit von mindestens einem Zehntausendstel (1/10000) gemessen, um eine Reihe H von Höhenmessungen zu erhalten; und
b) Werte, die in der im Schritt a) erhaltenen Reihe H umfasst sind, werden verwendet, um eine Reihe D von Durchsätzen gleichzusetzenden Mengen des Gases zu berechnen, die Volumenänderungen des Bades (2) im Laufe des vorbestimmten Zeitraums entsprechen.

2. Verfahren nach Anspruch 1, **dadurch gekennzeichnet, dass** im Schritt b) jede Menge aus der Reihe D gemäß einem Algorithmus berechnet wird, der die folgenden Schritte umfasst:
i) zwei zur Reihe H gehörende Höhenmessungen (3), die zwei Messzeitpunkten entsprechen, werden in jeweils zwei Volumen des Bades (2) umgerechnet;
ii) die Differenz zwischen den zwei unter i) erhaltenen Volumen wird durch die Dauer dividiert, die die Messzeitpunkte trennt, um den Flüssigkeitsdurchsatz zu erhalten, der während der die Zeitpunkte trennenden Dauer verbraucht wurde;
iii) wenn der unter ii) erhaltene Flüssigkeitsdurchsatz kleiner ist als eine vorbestimmte Schwelle, wird der Flüssigkeitsdurchsatz mit einem gegebenen Expansionsfaktor multipliziert, um die Menge des Gases, das vom Patienten verbraucht wurde, zu erhalten; und
iv) wenn der unter ii) erhaltene Flüssigkeitsdurchsatz größer oder gleich der vorbestimmten Schwelle ist, wird die verbrauchte Menge des Gases als Null betrachtet.

3. Verfahren nach einem der Ansprüche 1 oder 2, **dadurch gekennzeichnet, dass** das aus dem Kryobehälter (1) stammende Gas (4) überwiegend Sauerstoff umfasst.

4. Verfahren nach einem der Ansprüche 1 bis 3, **dadurch gekennzeichnet, dass** im Schritt a) die Höhe (3) mithilfe von mindestens einer Sonde (5) vom kapazitiven Typ gemessen wird.

5. Verfahren nach einem der Ansprüche 1 bis 4, **dadurch gekennzeichnet, dass** im Schritt a) die Höhe (3) in Zeitintervallen von regelmäßigen Zeiten zwischen 0,1 Sekunden und 1 Stunde, vorzugsweise zwischen 1 und 60 Sekunden und noch bevorzugter alle 10 Sekunden gemessen wird.

6. Verfahren nach einem der Ansprüche 1 bis 5, **dadurch gekennzeichnet, dass** es weiter einen Schritt c) umfasst, in dem die gesamte oder ein Teil der Reihe D von Mengen, die im Schritt b) erhalten wurden, über Funkwellen (6) an ein GPRS-Gateway (7), und anschließend über GPRS-Signale (13) an einen entfernten Server (8) gesendet werden.

7. Verfahren nach Anspruch 6, **dadurch gekennzeichnet, dass** im Schritt c) die gesamte oder ein Teil der Reihe D von Mengen, die im Schritt b) erhalten wurden, außerhalb der Zeitpunkte, zu denen Messungen im Schritt a) vorgenommen werden, über Funkwellen (6) gesendet werden.

8. Vorrichtung zum Nachverfolgen des Gasverbrauchs ab einem Kryobehälter (1), der das Gas in verflüssigter Form speichert, insbesondere für die Einhaltung, durch einen Patienten, einer Behandlung, die das Inhalieren des verbrauchten Gases (4) umfasst, wobei das Gas über ein Rohrleitungssystem (15) aus dem Kryobehälter (1) verbraucht wird, der ein Bad (2) des in flüssiger Form gespeicherten Gases umfasst, wobei das Bad ein bestimmtes Volumen aufweist, das einer bestimmten Flüssigkeitshöhe (3) entspricht, **dadurch gekennzeichnet, dass** sie umfasst:
- mindestens eine Messsonde (5), die dazu in der Lage und bestimmt ist, Primärsignale (9) zu erzeugen, die für die Flüssigkeitshöhe (3) des Bades (2) zu verschiedenen Zeitpunkten repräsentativ sind; und
- einen Digitalwandler (10), der konfiguriert ist, um die Primärsignale in Sekundärsignale (11) umzuwandeln, die für äquivalente verbrauchte Gasdurchsätze repräsentativ sind.

9. Vorrichtung nach Anspruch 8, **dadurch gekennzeichnet, dass** die Messsonde (5) vom kapazitiven Typ ist.

10. Vorrichtung nach einem der Ansprüche 8 oder 9, **dadurch gekennzeichnet, dass**:
- der Digitalwandler (10) mit dem Kryobehälter (1) verbunden und elektrisch an die Messsonde (5) angeschlossen ist; und
- die Vorrichtung einen Sender (12) umfasst, der elektrisch an den Digitalwandler (10) angeschlossen und dazu in der Lage und bestimmt ist, die Sekundärsignale (11) in ternäre Funksignale (6) zu transformieren.

11. Vorrichtung nach Anspruch 10, **dadurch gekennzeichnet, dass**:
- die Vorrichtung ein vom Kryobehälter (1) getrenntes GPRS-Gateway (7) umfasst, das dazu in der Lage und bestimmt ist, die ternären Funksignale (6) zu empfangen und dieselben in Form von quaternären Signalen (13) vom GPRS-Typ neu zu senden; und
- die Vorrichtung mindestens einen entfernten Server (8) umfasst, der dazu in der Lage und bestimmt ist, die quaternären Signale (13) vom GPRS-Typ zu empfangen und zu verarbeiten.

## Claims

1. Method for monitoring a flow of gas (4) consumed from a cryogenic reservoir (1) storing the gas in a liquefied form, said consumed gas being transported by a duct system (15) from the cryogenic reservoir (1), the reservoir (1) comprising at least one pool (2) of said gas stored in a liquid form, the pool (2) having a given volume corresponding to a certain height (3) of liquid, **characterised in that** it comprises at least the following steps:
a) at numerous instants during a predetermined period, said height (3) of liquid is measured with a relative accuracy of at least one ten thousandth (1/10000) to obtain a series H of height measurements; and
b) the values of the series H obtained at step a) are used to calculate a series D of quantities of said gas that can be likened to flows, corresponding to variations of the volume of said pool (2) during said predetermined period.

2. Method according to claim 1, **characterised in that**, at step b), each quantity of the series D is calculated with an algorithm that comprises the following steps:
i) two height (3) measurements belonging to said series H, corresponding to two measurement instants, are converted into respectively two volumes of said pool (2);
ii) the difference between the two volumes obtained at i) is divided by the period of time separating said measurement instants in order to obtain the flow of liquid consumed during the period of time separating said instants;
iii) if said flow of liquid obtained at ii) is below a predetermined threshold, said liquid flow is multiplied by a given expansion factor to obtain said quantity of said gas consumed by the patient; and
iv) if said flow of liquid obtained at ii) is greater than or equal to said predetermined threshold, the quantity of said consumed gas is considered to be nil.

3. Method according to any of the claims 1 or 2, **characterised in that** said gas (4) from said cryogenic reservoir (1) predominantly comprises oxygen.

4. Method according to any of claims 1 to 3, **characterised in that**, at step a), said height (3) is measured with at least one probe (5) of the capacitive type.

5. Method according to any of claims 1 to 4, **characterised in that**, at step a), said height (3) is measured at regular time intervals ranging from 0.1 second to 1 hour, preferably from 1 to 60 seconds, and even more preferably every 10 seconds.

6. Method according to any of claims 1 to 5, **characterised in that** it further comprises a step c) whereby all or part of the series D of quantities obtained at step b) are sent by radio waves (6) to a GPRS gateway (7) and then to a remote server (8) through GPRS signals (13).

7. Method according to claim 6, **characterised in that** at step c), all or part of the series D of quantities obtained at step b) are sent by radio waves (6) at moments other than the instants when the measurements of step a) are performed.

8. Device for monitoring the gas consumption from a cryogenic reservoir (1) storing the gas in a liquefied form, in particular for the observance by a patient of a treatment comprising the inhalation of the consumed gas (4), the gas being consumed through a duct system (15) from the cryogenic reservoir (1) comprising a pool (2) of said stored gas in a liquid form, said pool having a determined volume corresponding to a determined height (3) of liquid, **characterised in that** it comprises:
- at least one measurement probe (5) configured and intended to produce primary signals (9) representative of said height (3) of liquid in the pool (2) at different instants; and
- a digital converter (10) configured to convert said primary signals to secondary signals (11) representative of the equivalent consumed flows of said gas.

9. Device according to claim 8, **characterised in that** said measurement probe (5) is of the capacitive type.

10. Device according to any of claims 8 or 9 **characterised in that**:
- said digital converter (10) is secured to said cryogenic reservoir (1) and electrically connected to said measurement probe (5); and
- said device comprises a transmitter (12) electrically connected to said digital converter (10) and configured and intended to transform said secondary signals (11) into ternary (6) radio signals

11. Device according to claim 10, **characterised in that**:
- said device comprises a GPRS gateway (7) separate from said cryogenic reservoir (1), configured and intended to receive said ternary (6) radio signals and to retransmit them in the form of quaternary signals (13) of the GPRS type; and
- said device comprises at least one remote server (8) configured and intended to receive and process said quaternary signals (13) of the GPRS type.
